Europäisches Patentamt

European Patent Office

Office européen des brevets

(19)

(11) **EP 0 744 191 A2**

(12) **EUROPEAN PATENT APPLICATION**

(43) Date of publication:
27.11.1996 Bulletin 1996/48

(51) Int. Cl.$^6$: **A61N 5/06**

(21) Application number: **96303671.0**

(22) Date of filing: **23.05.1996**

(84) Designated Contracting States:
**BE CH DE DK ES FR GB GR IE IT LI LU NL PT SE**

(30) Priority: **23.05.1995 JP 123572/95**

(71) Applicant: **Numao, Takashi**
**Shinjuku-ku, Tokyo 161 (JP)**

(72) Inventor: **Numao, Takashi**
**Shinjuku-ku, Tokyo 161 (JP)**

(74) Representative: **Leale, Robin George**
**Frank B. Dehn & Co.,European Patent Attorneys,**
**179 Queen Victoria Street**
**London EC4V 4EL (GB)**

(54) **Ultraviolet light measuring sheet, and artificial tanning apparatus incorporating the same**

(57)     An ultraviolet light measuring sheet comprises a sheet base (50) and a photochromic layer (51) which changes its colour in accordance with the intensity of ultraviolet light. The ultraviolet light measuring sheet is advantageous in its simple structure, light weight, low cost, and easy handling and operation.

Fig. 1

EP 0 744 191 A2

Printed by Rank Xerox (UK) Business Services
2.13.8/3.4

## Description

The present invention relates to an ultraviolet light (UV) measuring sheet and an artificial tanning apparatus provided with UV measuring function which is used in, for example, suntan salons, sports clubs and hotels.

Generally, tanning apparatus has a light source, for example specific UVA tubes and fluorescent tubes, to radiate UVA (ultraviolet light A) with which the human skin is irradiated for a predetermined period of time, thereby achieving the effect of pigmentation of the skin, i.e. so-called tanning. When such UVA fluorescent tubes have been used for a certain period of time, the intensity of UVA radiation (strength of UV radiation) which they produce is decreased, to thereby lower the tanning effect thereof. However, the visible light (having longer wave length than UV) radiation of the UVA tubes is not decreased in intensity simultaneously with that of the UV radiation, and the tube thus has a longer lighting life than the UV radiation. Therefore, the life of the fluorescent tube is judged by the total duration of irradiation as monitored by a time measuring device disposed in the tanning apparatus, on the basis of the estimated life time given by the manufacturer of the UVA tube. Alternatively, the strength of the UVA radiation or the life of the UVA tube is measured by a light intensity meter with a UV filter. In the former case, when the total time of irradiation has passed the estimated life time, the UVA tubes are replaced by new ones, and in the latter case, when the measurement of UVA is judged as nil or of insufficient intensity for tanning, the UVA tubes need to be replaced.

However, the conventional UV measuring systems have drawbacks, residing in complicated structures, expensiveness, inconvenience to carry, and annoying measuring operation. Further, the estimated life time of the UVA tubes indicated by the manufacturers not only is inaccurate, but also varies within a significant range. So, there have been problems in that still useful UVA tubes are sometimes replaced by new ones, or tubes already producing unfavorably low UV radiation intensities are left unreplaced.

According to the present invention there is provided an ultraviolet light measuring sheet comprising a sheet base and an ultraviolet light-sensitive part disposed on the surface of the base, which part changes its colour in accordance with the intensity of ultraviolet light.

The UV measuring sheet may further comprise a coloured part exhibiting reference colours, which is disposed on the surface of the base for evaluating the level of colour change of the UV-sensitive part.

The UV-sensitive part may be composed of a photochromic material. Further, the part can be composed of a material such that, once it changes its colour, it retains the density of the new colour, or other kinds of materials whose colour density changes in accordance with the amount of absorbed UV (i.e. the integrated amount of the absorption = intensity of UV x absorption time ).

A UV measuring sheet according to the present invention has a simple structure and is light and inexpensive. Its UV-sensitive part changes colour in accordance with the intensity of irradiated UV. A UV measuring sheet having reference colours permits a user to judge whether or not the UV intensity is sufficient for efficient tanning, by comparing the colour of the UV-sensitive part with the reference colours.

A UV measuring sheet in which the UV-sensitive part is made of photochromic material, or a material which changes its colour after the completion of measurement, permits restoration of the colour of the UV-sensitive part to the original, or retention of the density of the changed colour of the UV-sensitive part, after the completion of the measurement. A UV measuring sheet in which the UV-sensitive part is made of a material which changes the density of its colour in accordance with the amount of absorbed UV, permits changing of the density of the colour of the UV-sensitive part in accordance with the amount of absorbed UV.

A tanning apparatus according to the present invention comprises a light source capable of radiating ultraviolet light, a light source supporting member arranged to support the light source, and a UV measuring sheet as set forth above located to receive UV light from the light source.

Some embodiments of the invention will now be described by way of example and with reference to the accompanying drawings, in which:

Fig. 1 is a perspective view of a UV measuring sheet according to the present invention;
Fig. 2 is a schematic perspective view of a tanning apparatus according to the invention;
Fig. 3 is a partial perspective view of a modified tanning apparatus according to the invention; and
Fig. 4 is a schematic perspective view of a table top tanning apparatus according to the invention.

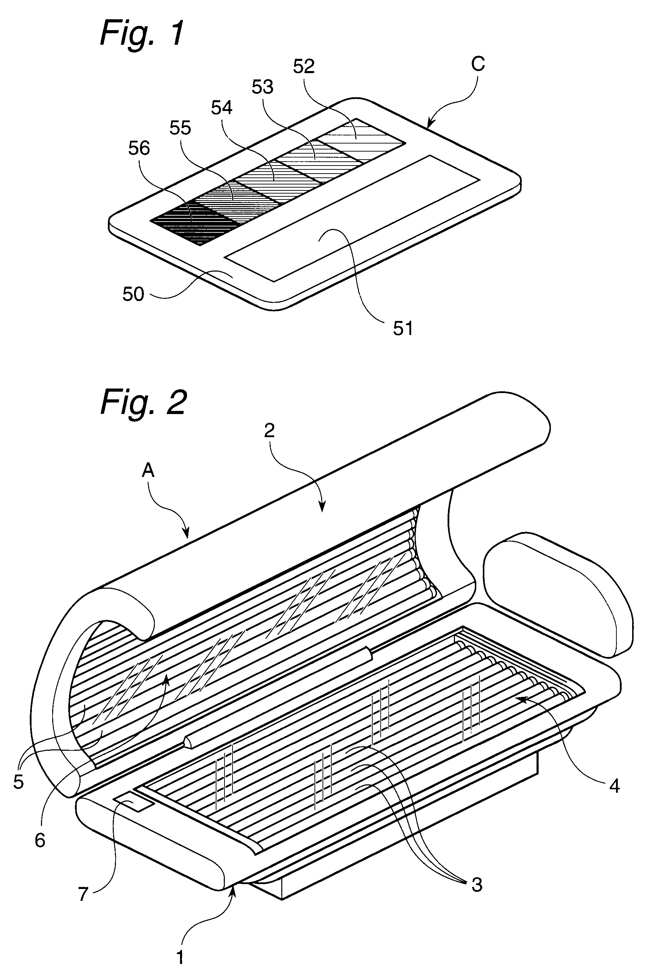

Fig. 1 is a perspective view of a UV measuring sheet C according to one embodiment of the present invention. The UV measuring sheet C has an approximately rectangularly shaped sheet (card) base 50. The base 50 is composed of a material such as a plastic, a metal, or a paper and shaped into a rectangle with a size as may be held in the palm. A UV-sensitive part, i.e. photochromic layer 51, which exhibits a reversible colour change upon absorption of UV is disposed on an upper surface of the base 50.

The photochromic layer 51 is formed of, for example, an organic material such as a spiropyran or ethylideneaniline, a system composed of silver halide, azobenzene, thioindigo, soda lime or titanium hydrochloride doped with oxides of divalent nickel or hexavalent molybdenum, or a system composed of calcium fluoride doped with cerium.

A coloured part exhibiting reference colours 52 to 56 of differing density corresponding to the density in the developed (changed) colour of the photochromic layer 51 in accordance with the intensity of UV, is disposed on another region of the upper surface of the base 50. In this embodiment, reference colour 52 has the lowest density and reference colour 56 does the highest density. For example, when the intensity of UV ranges from 10 mW/cm$^2$ to 25 mW/cm$^2$, the reference colour 52 indicates that the intensity of UV is from not less than 10 mW/cm$^2$ to less than 13 mW/cm$^2$ and is insufficient; the reference colour 53 indicates that the intensity of UV is from not less than 13 mW/cm$^2$ to less than 16 mW/cm$^2$ and is relatively lowered; the reference colour 54 indicates that the intensity of UV is from not less than to 16 mW/cm$^2$ to less than 19 mW/cm$^2$ and is sufficient; the reference colour 55 indicates that the intensity of UV is from not less than 19 mW/cm$^2$ to less than 22 mW/cm$^2$ and is fairly high; and the reference colour 56 indicates that the intensity of UV is from not less than 22 mW/cm$^2$ to not more than 25 mW/cm$^2$ and is high. In this embodiment, the number of the reference colours and the intensity of UV indicated by each reference colour in the coloured part may be varied.

In use of the UV measuring sheet C, the measurer puts the measuring sheet in the vicinity of the fluorescent tubes (UVA tubes) of the tanning apparatus (see Embodiment 2) to thereby irradiate the photochromic layer 51 with UV. Upon irradiation, the photochromic layer 51 changes its colour (colouring) in accordance with the intensity of UV, within a few minutes. The density of the colour of the photochromic layer 51 is compared with the densities of the coloured part exhibiting reference colours 52 to 56.

For example, if the density of the colour of the photochromic layer 51 is equivalent to that of the reference colour 53 of the coloured part, the intensity of UV is judged as being satisfactory. If the density of the colour of the photochromic layer 51 is lower than that of the reference colour 52 of the coloured part, the intensity of UV is judged as being unsatisfactory. In this situation, the fluorescent tubes should be replaced by new ones. Naturally, if the ultraviolet measuring sheet C is distanced from the fluorescent tubes, the colour of the photochromic layer 51 returns to the original colour.

Thus, in this embodiment, the UV measuring sheet C is light, through having the base 50 in the form of a sheet (card), has a simple structure so as to permit production at a lowered cost, and is easy to carry. The photochromic layer 51 changes its colour in accordance with the intensity of UV only by putting the UV measuring sheet C in the vicinity of the fluorescent tubes. So, the measurer can simply and easily measure the intensity of UV without conducting any special measuring operation.

Further, since the UV measuring sheet C is extremely light, it can be easily kept in an unchanged position, to thereby enhance the measuring accuracy. Besides, as the photochromic layer 51 is restored to its original colour after the measurement is completed, the UV measuring sheet C can be used repeatedly. Moreover, by comparing the colour of the photochromic layer 51 with the reference colours 52 to 56 of the coloured part, it can be judged whether or not the intensity of UV is sufficient.

In this embodiment, the number of reference colours 52 to 56 of the coloured part can be arbitrarily increased or decreased, and also the coloured part can be moved. The UV measuring sheet C may be shaped so as to have a circular or other form. Although the photochromic layer 51 is formed on the upper surface of the base 50 in this embodiment, the whole of the UV measuring sheet may be integrally formed of a photochromic material by a process comprising melt blending the photochromic material and performing injection or extrusion molding of the blend.

In an alternative embodiment the UV-sensitive part of the Fig. 1 device can be composed of a material which, having once changed its colour, retains the density of the new colour. Examples of such materials include a photosensitive material of the diazo type and a material obtained by immersing a known photosensitive material in an aqueous solution of an organic material (e.g., β-naphtholdisulfonic acid) or mineral oil which emits fluorescence upon irradiation with UV to thereby convert the UV-range sensitivity thereof to the fluorescence visible-range sensitivity.

The same effects as in the Fig. 1 embodiment can be obtained in this embodiment as well. In this embodiment, the UV measuring sheet must be replaced with a new one when the fluorescent tubes are replaced because once the UV-sensitive part changes its colour, it retains the density of the new colour.

In another alternative the UV-sensitive part of the Fig. 1 embodiment can be composed of a material whose colour density changes in accordance with the amount of absorbed ultraviolet. In the present specification, "amount of absorbed UV" means an integrated amount of absorption due to the UV irradiation and can be calculated from the formula: integrated amount of absorption = intensity of UV x absorption time . This function can be achieved by lowering the photosensitivity of the UV-sensitive part as much as possible. For example, the desired UV-sensitive part can be formed by coating an upper surface of a base with a solution of a diazonium compound, a coupler (an aromatic amine such as aniline and phenylenediamine, or a phenol compound such as phenol, p-cresol, α-naphthol, hydroquinone and fluorobrucine), an alkali developer (an ammonium salt such as an ammonium halide or ammonium carbonate) and a resin, and drying the coated base. In this structure, the resin absorbs part of the UV, so that the photosensitivity of the UV-sensitive part is lowered (the greater the proportion of the resin there is, the greater the decrease of the photosensitivity will be) to thereby retard the advance of colour change.

Further, as another means for lowering the photosensitivity of the UV-sensitive part as much as possible, the UV-sensitive part can for example be formed by first applying on an upper surface of a base a solution obtained by dissolving a diazonium compound and a binding agent such as polyvinyl alcohol in a solvent (e.g., water or alcohol) and drying to thereby form a sublayer and secondly coating the surface of the sublayer with a solution of a coupler (an aromatic amine such as aniline and phenylenediamine, or a phenol compound such as phenol, p-cresol, $\alpha$-naphthol, hydroquinone and fluorobrucine), an alkali developer (an ammonium salt such as an ammonium halide and ammonium carbonate) and a resin, and drying the coated base to thereby form an upper layer. In this structure, the greater the thickness of the upper layer containing the resin is, the lower the photosensitivity of the ultraviolet-sensitive part is.

The same effects as in the Fig. 1 embodiment can be achieved in this embodiment as well. Further, in this embodiment, the UV-sensitive part changes its density of colour in accordance with the amount of absorbed UV (i.e. integrated amount of absorption = intensity of UV x absorption time ). Therefore, in the measurement using the UV measuring sheet of this embodiment, the measuring object is limited to a single tanning apparatus. That is, a measuring sheet in use for one tanning apparatus cannot be used on another.

Fig. 2 is a perspective view of a floor tanning apparatus A provided with UV measuring function. The tanning apparatus A has a bed portion 1 (first light source supporting member) and a roof portion 2 (second light source supporting member). The bed portion 1 is shaped into a planar approximately rectangular form and has such a planar area that a human can lie at full length on the upper surface thereof. The bed portion 1 is provided along its longitudinal direction with a light source, for example a plurality of long cylindrical fluorescent tubes 3. A translucent cover plate 4, which is made of an acrylic resin for example, is provided above the fluorescent tubes 3.

The roof portion 2 is connected to a long side of the bed portion 1 in such a manner that it can be freely opened and closed and is molded so as to have a circular-arc-shaped cross section. The roof portion 2 is provided along its longitudinal direction with a light source, for example a plurality of long cylindrical fluorescent tubes 5. The inner circumferential surface of the roof portion 2 is covered with a translucent plate 6 made of an acrylic resin for example. The fluorescent tubes 3 and 5 may be surrounded with a reflection member (not shown).

A UV measuring sheet 7 is stuck by means of an adhesive on a zone irradiated with UV radiated by the fluorescent tubes 3 and 5, i.e. the upper surface of a corner of the bed portion 1 in this embodiment. The UV measuring sheet 7 has substantially the same construction as that of the UV measuring sheet C employed in the Fig. 1 embodiment except that the thickness of the base is minimized to have the form of a film (label), so that the rigidity of the base is reduced to become easily deformable in accordance with the configuration of the object on which the UV measuring sheet 7 is stuck, with the result that the adhesion of the UV measuring sheet 7 to the object is improved.

When the fluorescent tubes 3 and 5 are energised by operating a switch (not shown) of the tanning apparatus A, UV having a wavelength set in the UVA region (ranging from 320 to 400 nm from the viewpoint of user safety and tanning efficiency) is radiated from the fluorescent tubes 3 and 5. When UV reaches the photochromic layer of the UV measuring sheet 7, the photochromic layer changes its colour (colouring) in accordance with the intensity of UV, within a few minutes. The colour of the photochromic layer is compared with the reference colours of the coloured part, and judgment is made in the same manner as in the Fig. 1 embodiment.

The same effects as in the Fig. 1 embodiment (except that the stuck UV measuring sheet 7 cannot be carried) can be achieved in this embodiment as well. The sticking of the UV measuring sheet 7 on the bed portion 1 has another effect, namely that the measuring position, i.e., the distance between the UV measuring sheet 7 and the fluorescent tubes, is being always unchanged, to thereby further enhance the measuring accuracy.

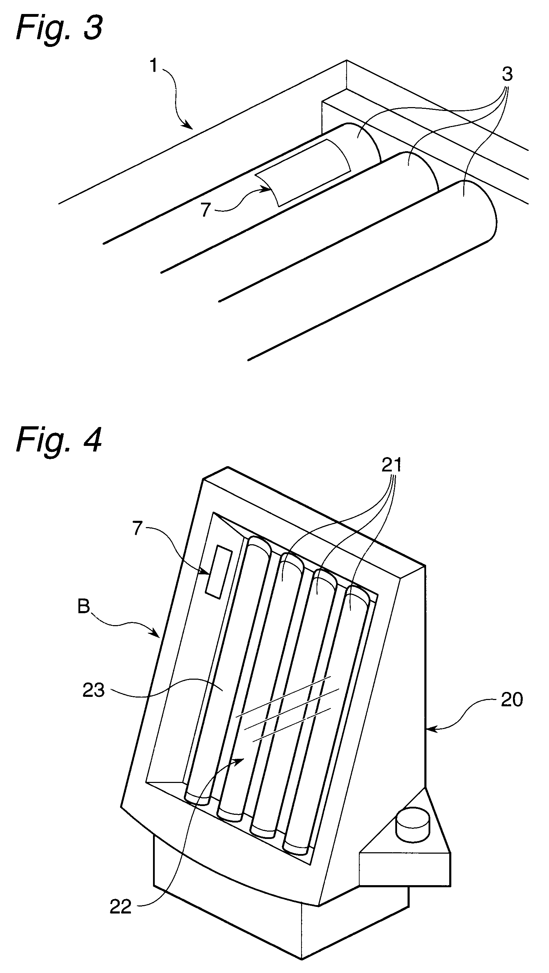

Fig. 3 shows an embodiment in which the UV measuring sheet 7 is stuck at a different position. In this embodiment, the UV measuring sheet 7 is directly stuck on the surface of a fluorescent tube 3, near to one end. The same effects as in the Fig. 2 embodiment can be achieved in this embodiment as well.

Although the UV measuring sheet 7 is stuck on the bed portion 1 in the Fig. 2 embodiment and on a fluorescent tube 3 in the Fig. 3 embodiment, the same effects can be achieved by sticking the UV measuring sheet 7 on the inner circumferential surface of the roof portion 2 or on the surface of one of the fluorescent tubes 5. Further, the same effects can be achieved by sticking the UV measuring sheet 7 on the surface of the translucent plate 4 or 6. Still further, the same effects can be achieved by sticking the UV measuring sheet 7 on a reflection member (not shown) surrounding the fluorescent tubes 3 or 5. Thus the position where the UV measuring sheet 7 is to be stuck is not limited as long as it can be viewed by the measurer from the vicinity of the tanning apparatus A and is in the area irradiated with UV radiated from the fluorescent tubes 3 and 5, namely about 10 cm from the tubes.

Fig. 4 shows a table top tanning apparatus B. The tanning apparatus B comprises an apparatus body 20 (light source supporting member) and a light source, e.g., fluorescent tubes 21 longitudinally arranged on the front of the apparatus body 20. The front of the apparatus body 20 is covered with a translucent plate 22 made of an acrylic resin for example. A reflection member 23 capable of reflecting radiated UV is provided in the vicinity of the fluorescent tubes 21. The UV measuring sheet 7 is stuck on the reflection member 23. The same effects as in the Fig. 2 embodiment can be achieved in this embodiment as well.

In other alternatives, the UV measuring sheet 7 can be stuck on a tanning apparatus of the floor standing type which is provided with castors, or on a tanning apparatus of the desk stand type which is provided with castors. The shape of the light source of the tanning apparatus can be spherical, annular or flat. Further, the UV-sensitive part of each of the UV measuring sheets of the embodiments of Figs. 2, 3 and 4 can have the same construction as in the previously described modifications of the Fig. 1 embodiment.

## Claims

1. An ultraviolet light measuring sheet (C) comprising a sheet base (50) and an ultraviolet light-sensitive part (51) disposed on the surface of the base, which part changes its colour in accordance with the intensity of ultraviolet light.

2. A device as claimed in claim 1, further comprising a coloured part (52-56) exhibiting reference colours, which is disposed on the surface of the base (50) for evaluating the level of colour change of the ultraviolet light-sensitive part (51).

3. A device as claimed in claim 1 or 2, wherein the ultraviolet light-sensitive part (51) is composed of a photochromic material.

4. A device as claimed in claim 1 or 2, wherein the ultraviolet light-sensitive part (51) is composed of a material which, once it changes its colour, retains the density of the new colour.

5. An ultraviolet light measuring sheet (C) comprising a sheet base (5) and an ultraviolet light-sensitive part (51) disposed on the surface of the base, which part is composed of a material whose colour density changes in accordance with the amount of absorbed ultraviolet light.

6. A tanning apparatus comprising a light source (3,5;21) capable of radiating ultraviolet light, a light source supporting member (1,2;20) arranged to support the light source, and an ultraviolet light measuring sheet (C) as claimed in any of claims 1 to 5 located to receive ultraviolet light from said light source.

*Fig. 1*

*Fig. 2*

## Fig. 3

## Fig. 4